# EUROPEAN PATENT APPLICATION

(11) **EP 4 609 864 A1**
(43) Date of publication of application: **03.09.2025**
(21) Application number: 23881939.5
(22) Date of filing: 27.10.2023
(51) Int. Cl.: A61K 31/519, A61K 9/12, A61K 9/19, A61K 9/72, A61P 11/06, A61P 11/08, A61P 13/12, A61P 3/10, A61P 1/04, A61P 27/02

(54) **PHARMACEUTICAL COMPOSITION CONTAINING ISOQUINOLINONE COMPOUND, AND PREPARATION METHOD THEREFOR**

(30) Priority: 28.10.2022 CN 202211335157
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: YANG, Yi, Shanghai 200245 (CN); WANG, Haifeng, Shanghai 200245 (CN); ZHOU, Yin, Shanghai 200245 (CN); WANG, Pingping, Shanghai 200245 (CN); ZHOU, Xueping, Shanghai 200245 (CN); WANG, Jingwen, Shanghai 200245 (CN)
(74) Representative: Regimbeau
(86) International application number: PCT/CN2023/126961
(87) International publication number: WO 2024/088364

(57) **Abstract**

The present disclosure relates to a pharmaceutical composition containing an isoquinolinone compound, and to a preparation method therefor. Specifically, the present disclosure provides a pharmaceutical composition containing 9,10-dimethoxy-2-[[2-(2-oxo-imidazoline-1-yl)-ethyl]-(2,4,6-trimethyl-phenyl)-amino]-6,7-dihydro-pyrimidinyl[6,1-a]-isoquinolin-4-one or a pharmaceutically acceptable salt thereof, and polysorbate 80. The pharmaceutical composition of the present disclosure exhibits good physical and chemical stability after being stored for several months.

## Description

### TECHNICAL FIELD

The disclose pertains to the field of pharmaceutical formulations and relates to a pharmaceutical composition comprising an isoquinolinone compound and a preparation method therefor.

### BACKGROUND

A new molecule with inhibitory activity against both PDE3 and PDE4 can offer the bronchodilatory effects of β-adrenergic receptor agonists and the anti-inflammatory effects of inhaled glucocorticoids. The two complementary targeting abilities can achieve a better effect than a single one. For example, RPL554 (9,10-dimethoxy-2-(2,4,6-trimethylphenylimino)-3-(N-carbamoyl-2-aminoethyl)-3,4,6,7-tetrahydro-2H-pyrimido[6,1-α]isoquinolin-4-one) is a PDE3/PDE4 dual-target inhibitor disclosed in WO00/58308. Recent phase II clinical data show that the drug can significantly improve bronchodilation and symptoms in patients with chronic obstructive pulmonary disease, and that the drug was well tolerated and did not cause noticeable adverse events; for example, problems with the heart, nausea, and diarrhea were all mild. The safety of the drug and its "limited systemic exposure" are encouraging.

9,10-Dimethoxy-2-[[2-(2-oxo-imidazolidin-1-yl)-ethyl]-(2,4,6-trimethyl-phenyl)-amino]-6,7-dihydro-pyrimido[6,1-*a*]-isoquinolin-4-one is a novel PDE3/4 inhibitor having more excellent inhibitory activity against PDE3 and PDE4:

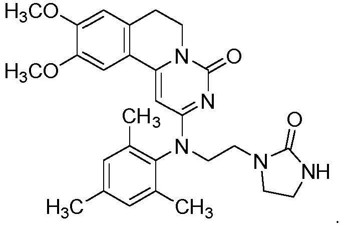

To adapt the new PDE3/4 inhibitor for administration, ensure its stability during storage and subsequent use, and enable it to achieve a better effect, it is necessary to develop a pharmaceutical (formulation) composition that is more suitable for administration.

### SUMMARY

The disclose provides a pharmaceutical composition comprising 9,10-dimethoxy-2-[[2-(2-oxo-imidazolidin-1-yl)-ethyl]-(2,4,6-trimethyl-phenyl)-amino]-6,7-dihydro-pyrimido[6,1-*a*]-isoquinolin-4-one or a pharmaceutically acceptable salt thereof as an active ingredient, and polysorbate 80.

The active ingredient in the pharmaceutical composition of the disclose is at a concentration of 0.01 mg/mL to 40 mg/mL, including but not limited to 0.01 mg/mL, 0.02 mg/mL, 0.03 mg/mL, 0.04 mg/mL, 0.05 mg/mL, 0.1 mg/mL, 0.3 mg/mL, 0.5 mg/mL, 0.7 mg/mL, 0.9 mg/mL, 1.1 mg/mL, 1.3 mg/mL, 1.5 mg/mL, 1.7 mg/mL, 1.9 mg/mL, 2.1 mg/mL, 2.3 mg/mL, 2.5 mg/mL, 2.7 mg/mL, 2.9 mg/mL, 3.1 mg/mL, 3.3 mg/mL, 3.5 mg/mL, 3.7 mg/mL, 3.9 mg/mL, 4.1 mg/mL, 4.3 mg/mL, 4.5 mg/mL, 4.7 mg/mL, 4.9 mg/mL, 5.1 mg/mL, 5.3 mg/mL, 5.5 mg/mL, 5.7 mg/mL, 5.9 mg/mL, 6.1 mg/mL, 6.3 mg/mL, 6.5 mg/mL, 6.7 mg/mL, 6.9 mg/mL, 7.1 mg/mL, 7.3 mg/mL, 7.5 mg/mL, 7.7 mg/mL, 7.9 mg/mL, 8.1 mg/mL, 8.3 mg/mL, 8.5 mg/mL, 8.7 mg/mL, 8.9 mg/mL, 9.1 mg/mL, 9.3 mg/mL, 9.5 mg/mL, 9.7 mg/mL, 9.9 mg/mL, 10.1 mg/mL, 10.3 mg/mL, 10.5 mg/mL, 10.7 mg/mL, 10.9 mg/mL, 11.1 mg/mL, 11.3 mg/mL, 11.5 mg/mL, 11.7 mg/mL, 11.9 mg/mL, 12.1 mg/mL, 12.3 mg/mL, 12.5 mg/mL, 12.7 mg/mL, 12.9 mg/mL, 13.1 mg/mL, 13.3 mg/mL, 13.5 mg/mL, 13.7 mg/mL, 13.9 mg/mL, 14.1 mg/mL, 14.3 mg/mL, 14.5 mg/mL, 14.7 mg/mL, 14.9 mg/mL, 15.1 mg/mL, 15.3 mg/mL, 15.5 mg/mL, 15.7 mg/mL, 15.9 mg/mL, 16.1 mg/mL, 16.3 mg/mL, 16.5 mg/mL, 16.7 mg/mL, 16.9 mg/mL, 17.1 mg/mL, 17.3 mg/mL, 17.5 mg/mL, 17.7 mg/mL, 17.9 mg/mL, 18.1 mg/mL, 18.3 mg/mL, 18.5 mg/mL, 18.7 mg/mL, 18.9 mg/mL, 19.1 mg/mL, 19.3 mg/mL, 19.5 mg/mL, 19.7 mg/mL, 19.9 mg/mL, 20.0 mg/mL, or a value between any two of these numerical values.

In some embodiments, the active ingredient in the pharmaceutical composition is at a concentration of 0.05 mg/mL to 20 mg/mL.

Further, the pharmaceutical composition of the disclose further comprises a buffer selected from the group consisting of a citrate buffer, a phosphate buffer, and an acetate buffer. In some embodiments, the buffer is selected from the group consisting of a phosphate buffer. In some other embodiments, the buffer is selected from the group consisting of a sodium dihydrogen phosphate-disodium hydrogen phosphate buffer. In some other embodiments, the buffer is selected from the group consisting of a sodium acetate-acetic acid buffer.

In an optional embodiment, the buffer has a concentration of 1 mM to 50 mM, including but not limited to 1 mM, 2 mM, 3 mM, 4 mM, 5 mM, 6 mM, 7 mM, 8 mM, 9 mM, 10 mM, 11 mM, 12 mM, 13 mM, 14 mM, 15 mM, 16 mM, 17 mM, 18 mM, 19 mM, 20 mM, 21 mM, 22 mM, 23 mM, 24 mM, 25 mM, 26 mM, 27 mM, 28 mM, 29 mM, 30 mM, 31 mM, 32 mM, 33 mM, 34 mM, 35 mM, 36 mM, 37 mM, 38 mM, 39 mM, 40 mM, 41 mM, 42 mM, 43 mM, 44 mM, 45 mM, 46 mM, 47 mM, 48 mM, 49 mM, 50 mM, or a value between any two of these numerical values.

In some embodiments, the buffer has a concentration of 5 mM to 30 mM.

In another aspect, the polysorbate 80 in the pharmaceutical composition provided by some embodiments is at a concentration of 0.05 mg/mL to 5.0 mg/mL, including but not limited to 0.05 mg/mL, 0.06 mg/mL, 0.07 mg/mL, 0.08 mg/mL, 0.09 mg/mL, 0.1 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, 1.0 mg/mL, 1.1 mg/mL, 1.2 mg/mL, 1.3 mg/mL, 1.4 mg/mL, 1.5 mg/mL, 1.6 mg/mL, 1.7 mg/mL, 1.8 mg/mL, 1.9 mg/mL, 2.0 mg/mL, 2.1 mg/mL, 2.2 mg/mL, 2.3 mg/mL, 2.4 mg/mL, 2.5 mg/mL, 2.6 mg/mL, 2.7 mg/mL, 2.8 mg/mL, 2.9 mg/mL, 3.0 mg/mL, 3.1 mg/mL, 3.2 mg/mL, 3.3 mg/mL, 3.4 mg/mL, 3.5 mg/mL, 3.6 mg/mL, 3.7 mg/mL, 3.8 mg/mL, 3.9 mg/mL, 4.0 mg/mL, 4.1 mg/mL, 4.2 mg/mL, 4.3 mg/mL, 4.4 mg/mL, 4.5 mg/mL, 4.6 mg/mL, 4.7 mg/mL, 4.8 mg/mL, 4.9 mg/mL, 5 mg/mL, or a value between any two of these numerical values.

In some embodiments, the polysorbate 80 in the pharmaceutical composition is at a concentration of 0.08 mg/mL to 0.5 mg/mL.

Further, the pharmaceutical composition of the disclose has a pH of 3.0 to 8.0, including but not limited to 3.0, 4.0, 5.0, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, or a value between any two of these numerical values. In some embodiments, the pharmaceutical composition has a pH of 6.0 to 7.0.

In another aspect, in some embodiments, the active ingredient in the pharmaceutical composition is present as particles, and the active ingredient particles have a particle size D90 of less than 7 µm that is selected from the group consisting of, but not limited to, 1.0 µm, 1.1 µm, 1.2 µm, 1.3 µm, 1.4 µm, 1.5 µm, 1.6 µm, 1.7 µm, 1.8 µm, 1.9 µm, 2.0 µm, 2.1 µm, 2.2 µm, 2.3 µm, 2.4 µm, 2.5 µm, 2.6 µm, 2.7 µm, 2.8 µm, 2.9 µm, 3.0 µm, 3.1 µm, 3.2 µm, 3.3 µm, 3.4 µm, 3.5 µm, 3.6 µm, 3.7 µm, 3.8 µm, 3.9 µm, 4.0 µm, 4.1 µm, 4.2 µm, 4.3 µm, 4.4 µm, 4.5 µm, 4.6 µm, 4.7 µm, 4.8 µm, 4.9 µm, 5.0 µm, and a value between any two of these numerical values.

In some embodiments, the active ingredient particles in the pharmaceutical composition have a particle size D90 of 2.0 µm to 4.0 µm.

In some embodiments, the active ingredient particles in the pharmaceutical composition have a particle size D90 of 2.0 µm to 3.5 µm.

In some embodiments, the active ingredient particles in the pharmaceutical composition have a particle size D90 of 3.0 µm or 3.2 µm.

In some other embodiments, the active ingredient particles in the pharmaceutical composition have a particle size D50 of 1.0 µm, 1.1 µm, 1.2 µm, 1.3 µm, 1.4 µm, 1.5 µm, 1.6 µm, 1.7 µm, 1.8 µm, 1.9 µm, 2.0 µm, 2.1 µm, 2.2 µm, 2.3 µm, 2.4 µm, 2.5 µm, 2.6 µm, 2.7 µm, 2.8 µm, 2.9 µm, 3.0 µm, 3.1 µm, 3.2 µm, 3.3 µm, 3.4 µm, 3.5 µm, 3.6 µm, 3.7 µm, 3.8 µm, 3.9 µm, 4.0 µm, or a value between any two of these numerical values. In some embodiments, the active ingredient particles in the pharmaceutical composition have a particle size D50 of 2.0 µm to 3.0 µm.

In some embodiments, the active ingredient particles in the pharmaceutical composition have a particle size D50 of 1.0 µm to 2.0 µm.

In some embodiments, the active ingredient particles in the pharmaceutical composition have a particle size D50 of 1.6 µm or 1.8 µm.

In another aspect, the active ingredient particles in the pharmaceutical composition provided by some embodiments have a particle size D10 of 0.1 µm, 0.2 µm, 0.3 µm, 0.4 µm, 0.5 µm, 0.6 µm, 0.7 µm, 0.8 µm, 0.9 µm, or a value between any two of these numerical values.

In another aspect, the average particle size is represented based on volume. The active ingredient particles in the pharmaceutical composition provided by some embodiments have a particle size D [4, 3] of 1.0 µm to 4 µm, including but not limited to 1.0 µm, 1.1 µm, 1.2 µm, 1.3 µm, 1.4 µm, 1.5 µm, 1.6 µm, 1.7 µm, 1.8 µm, 1.9 µm, 2.0 µm, 2.1 µm, 2.2 µm, 2.3 µm, 2.4 µm, 2.5 µm, 2.6 µm, 2.7 µm, 2.8 µm, 2.9 µm, 3.0 µm, 3.1 µm, 3.2 µm, 3.3 µm, 3.4 µm, 3.5 µm, 3.6 µm, 3.7 µm, 3.8 µm, 3.9 µm, 4.0 µm, or a value between any two of these numerical values.

In some embodiments, the active ingredient particles in the pharmaceutical composition have a particle size D [4, 3] of 1.6 µm or 1.7 µm.

In another aspect, the pharmaceutical composition of the disclose further comprises a sorbitan fatty acid ester.

In some embodiments, the sorbitan fatty acid ester in the pharmaceutical composition is at a concentration of 0.01 mg/mL to 0.5 mg/mL, including but not limited to 0.01 mg/mL, 0.02 mg/mL, 0.04 mg/mL, 0.06 mg/mL, 0.08 mg/mL, 0.1 mg/mL, 0.2 mg/mL, 0.3 mg/mL, 0.4 mg/mL, 0.5 mg/mL, or a value between any two of these numerical values.

In another aspect, the disclose also provides a pharmaceutical composition comprising particles of 9,10-dimethoxy-2-[[2-(2-oxo-imidazolidin-1-yl)-ethyl]-(2,4,6-trimethylphenyl)-amino]-6,7-dihydro-pyrimido[6,1-*a*]-isoquinolin-4-one or a pharmaceutically acceptable salt thereof as an active ingredient, wherein the active ingredient particles have a particle size D90 of less than 7 µm.

In some embodiments, the active ingredient particles in the pharmaceutical composition have a particle size D90 of 2.0 µm to 4.0 µm, such as 3.0 µm or 3.2 µm.

In some embodiments, the active ingredient particles in the pharmaceutical composition have a particle size D50 of 1.6 µm or 1.8 µm.

In some embodiments, the active ingredient particles in the pharmaceutical composition have a particle size D [4, 3] of 1.6 µm or 1.7 µm.

Further, the aforementioned pharmaceutical composition further comprises a tonicity modifier. In some embodiments, the tonicity modifier in the pharmaceutical composition is sodium chloride.

In some embodiments, the tonicity modifier in the pharmaceutical composition is at a concentration of 2 mg/mL to 8 mg/mL, including but not limited to 2.0 mg/mL, 2.2 mg/mL, 2.4 mg/mL, 2.6 mg/mL, 2.8 mg/mL, 3.0 mg/mL, 3.2 mg/mL, 3.4 mg/mL, 3.6 mg/mL, 3.8 mg/mL, 4.0 mg/mL, 4.2 mg/mL, 4.4 mg/mL, 4.6 mg/mL, 4.8 mg/mL, 5.0 mg/mL, 5.2 mg/mL, 5.4 mg/mL, 5.6 mg/mL, 5.8 mg/mL, 6.0 mg/mL, 6.2 mg/mL, 6.4 mg/mL, 6.6 mg/mL, 6.8 mg/mL, 7.0 mg/mL, 7.2 mg/mL, 7.4 mg/mL, 7.6 mg/mL, 7.8 mg/mL, 8.0 mg/mL, or a value between any two of these numerical values.

In some embodiments, the pharmaceutical composition comprises 0.05 mg/mL to 40 mg/mL 9,10-dimethoxy-2-[[2-(2-oxo-imidazolidin-1-yl)-ethyl]-(2,4,6-trimethylphenyl)-amino]-6,7-dihydro-pyrimido[6,1-*a*]-isoquinolin-4-one or a pharmaceutically acceptable salt thereof as an active ingredient, and 0.05 mg/mL to 5.0 mg/mL polysorbate 80.

In some embodiments, the pharmaceutical composition comprises 0.05 mg/mL to 40 mg/mL 9,10-dimethoxy-2-[[2-(2-oxo-imidazolidin-1-yl)-ethyl]-(2,4,6-trimethylphenyl)-amino]-6,7-dihydro-pyrimido[6,1-*a*]-isoquinolin-4-one or a pharmaceutically acceptable salt thereof as an active ingredient, and 0.05 mg/mL to 5.0 mg/mL polysorbate 80, wherein the active ingredient is present as particles.

In some embodiments, the pharmaceutical composition comprises:
a) 0.05 mg/mL to 40 mg/mL 9,10-dimethoxy-2-[[2-(2-oxo-imidazolidin-1-yl)-ethyl]-(2,4,6-trimethyl-phenyl)-amino]-6,7-dihydro-pyrimido[6,1-*a*]-isoquinolin-4-one or a pharmaceutically acceptable salt thereof as an active ingredient,
b) 0.05 mg/mL to 5.0 mg/mL polysorbate 80, and
c) a 1 mM to 50 mM buffer.

In some embodiments, the pharmaceutical composition comprises:
a) 0.05 mg/mL to 40 mg/mL 9,10-dimethoxy-2-[[2-(2-oxo-imidazolidin-1-yl)-ethyl]-(2,4,6-trimethyl-phenyl)-amino]-6,7-dihydro-pyrimido[6,1-*a*]-isoquinolin-4-one or a pharmaceutically acceptable salt thereof as an active ingredient,
b) 0.05 mg/mL to 5.0 mg/mL polysorbate 80, and
c) a 1 mM to 50 mM buffer, wherein the active ingredient is present as particles.

In some embodiments, the pharmaceutical composition comprises:
a) 0.05 mg/mL to 40 mg/mL 9,10-dimethoxy-2-[[2-(2-oxo-imidazolidin-1-yl)-ethyl]-(2,4,6-trimethyl-phenyl)-amino]-6,7-dihydro-pyrimido[6,1-*a*]-isoquinolin-4-one or a pharmaceutically acceptable salt thereof as an active ingredient,
b) 0.05 mg/mL to 5.0 mg/mL polysorbate 80, and
c) a 1 mM to 50 mM buffer, wherein the active ingredient is present as particles having a particle size D90 of less than 7 µm.

In some embodiments, the pharmaceutical composition comprises:
a) 0.05 mg/mL to 40 mg/mL 9,10-dimethoxy-2-[[2-(2-oxo-imidazolidin-1-yl)-ethyl]-(2,4,6-trimethyl-phenyl)-amino]-6,7-dihydro-pyrimido[6,1-*a*]-isoquinolin-4-one or a pharmaceutically acceptable salt thereof as an active ingredient,
b) 0.05 mg/mL to 5.0 mg/mL polysorbate 80,
c) a 1 mM to 50 mM buffer, and
d) 0.01 mg/mL to 0.5 mg/mL sorbitan fatty acid ester, wherein the active ingredient is present as particles having a particle size D90 of less than 7 µm.

The pharmaceutically acceptable salt of the active ingredient of the disclose includes, but is not limited to, a fumarate, a sulfate, and a hydrochloride. In some embodiments, the pharmaceutically acceptable salt is a fumarate.

In some embodiments, the pharmaceutical composition of the disclose is suitable for administration by a nebulizer. For example, the aforementioned pharmaceutical composition is delivered to the respiratory tract via a special device to exert local or systemic effects.

The disclose also provides a method for preparing the aforementioned pharmaceutical composition, the method comprising a step of grinding, wet grinding, homogenizing, precipitating, or shearing 9,10-dimethoxy-2-[[2-(2-oxo-imidazolidin-1-yl)-ethyl]-(2,4,6-trimethyl-phenyl)-amino]-6,7-dihydro-pyrimido[6,1-*a*]-isoquinolin-4-one or the pharmaceutically acceptable salt thereof.

In some embodiments, for process and economic reasons, the active ingredient is usually co-ground with all or a portion of the polysorbate 80 to prepare a concentrated suspension of the active ingredient, and the concentrated suspension is then diluted to obtain a suitable dilute suspension. The remaining portion of the surface stabilizer can be mixed with the aforementioned composition during dilution, thereby maintaining the stability of the diluted liquid. In another aspect, in an example of the disclose, in order to obtain D90 particles that meet conditions, particles having a suitable particle size are obtained by micronization, and after the particles are mixed with the aforementioned polysorbate 80, the mixture is ground and made into a suspension.

In an optional embodiment, the aforementioned preparation method further comprises a step of mixing with a buffer or/and a tonicity modifier.

In another aspect, the pharmaceutical compositions of the disclose may be further lyophilized to obtain a solid composition, such as a freeze-dried powder. In some embodiments, the pharmaceutical composition is stored in a vial.

In some embodiments, the lyophilization comprises steps of pre-freezing, primary drying, and secondary drying in sequence.

The disclose also provides a freeze-dried composition obtained by lyophilizing the aforementioned pharmaceutical composition. Further, the freeze-dried composition is reconstructed or reconstituted in a liquid medium to obtain the pharmaceutical composition, and the liquid medium used for reconstruction or reconstitution is selected from the group consisting of water for injection.

The disclose also provides a pharmaceutical composition consisting of the following ingredients, a freeze-dried composition prepared from the pharmaceutical composition, and a reconstituted solution obtained by further reconstructing or reconstituting the freeze-dried composition in a liquid medium, and the pharmaceutical composition comprises 9,10-dimethoxy-2-[[2-(2-oxo-imidazolidin-1-yl)-ethyl]-(2,4,6-trimethylphenyl)-amino]-6,7-dihydro-pyrimido[6,1-*a*]-isoquinolin-4-one or a pharmaceutically acceptable salt thereof.

In another aspect, the disclose also provides use of the aforementioned pharmaceutical composition, freeze-dried composition, or reconstituted solution in preparing a medicament for preventing and/or treating asthma, obstructive pulmonary disease, sepsis, nephritis, diabetes, allergic rhinitis, allergic conjunctivitis, ulcerative enteritis, or rheumatism, preferably obstructive pulmonary disease.

The disclose also provides a pharmaceutical composition for use in a medicament for preventing and/or treating asthma, obstructive pulmonary disease, sepsis, nephritis, diabetes, allergic rhinitis, allergic conjunctivitis, ulcerative enteritis, or rheumatism, preferably obstructive pulmonary disease.

### Terminology

"Buffer" refers to a buffer that resists changes in pH by the action of its acid-base conjugate components. Examples of buffers that control the pH within an appropriate range include, but are not limited to, citrates and phosphates.

"Phosphate buffer" is a buffer containing phosphate ions. Examples of phosphate buffers include disodium hydrogen phosphate-sodium dihydrogen phosphate, disodium hydrogen phosphate-potassium dihydrogen phosphate, and the like, and a preferred phosphate buffer is disodium hydrogen phosphate-sodium dihydrogen phosphate.

"Citrate buffer" is a buffer containing acetate ions. Examples of citrate buffers include citric acid-sodium citrate and the like. A preferred citrate buffer is a citric acid-sodium citrate buffer.

"Acetate buffer" is a buffer containing acetate ions. Examples of acetate buffers include acetic acid-sodium acetate and the like. A preferred acetate buffer is an acetic acid-sodium acetate buffer.

"Pharmaceutical composition" refers to a mixture containing one or more of the compounds described herein or physiologically/pharmaceutically acceptable salts or prodrugs thereof, and other chemical components, wherein the other components are, for example, physiologically/pharmaceutically acceptable carriers and excipients. The purpose of the pharmaceutical composition is to maintain the stability of the active ingredient and promote the administration to an organism, which facilitates the absorption of the active ingredient, thereby exerting biological activity. As used herein, "pharmaceutical composition" and "formulation" are not mutually exclusive.

"Freeze-dried formulation" refers to a formulation or a pharmaceutical composition obtained by lyophilizing a pharmaceutical composition in liquid or solution form or a solution formulation *in vacuo.*

As used herein, "D10" refers to the particle size at which the cumulative particle size distribution percentage of a sample reaches 10%. "D50" refers to the particle size at which the cumulative particle size distribution percentage of a sample reaches 50%. "D90" refers to the particle size at which the cumulative particle size distribution percentage of a sample reaches 90%.

As used herein, "D [4, 3]" represents the average particle size based on volume. Its full name is "mass to volume average particle size", or it is simply referred to as the "volume average particle size". It is calculated by taking the average of the particle size values at the two ends of each particle size interval, multiplying the average by the particle size distribution percentage corresponding to that interval, and then summing up these products. That is, D[4,3] = (f1·D1 + f2·D2 + f3·D3 +...).

All content percentages (%) of the substances in the disclose refer to weight-to-volume ratios. As used herein, "weight-to-volume ratio" refers to the weight (in g) of the ingredient contained in every 100 mL of the liquid system, i.e., g/100 mL.

The numerical values in the disclose are instrument measurements and have a certain degree of error. Generally, ±10% is a reasonable margin of error. It is certainly necessary to consider the context in which the numerical values are used; for example, the numerical value of the particle size of the active ingredient after the measurement has a margin of error of no greater than ±10%; the margin of error may be ±9%, ±8%, ±7%, ±6%, ±5%, ±4%, ±3%, ±2%, or ±1%, preferably ±5%.

Typical acceptable criteria for stability of the disclose are as follows: as measured by HPLC, the content change during storage is generally no greater than about 10%; preferably, no greater than about 5% of the active ingredient degrades. HPLC conditions: Gradient elution is performed, with octadecylsilane-bonded silica gel as a filler and a phosphate and triethylamine phosphate buffer/acetonitrile as a mobile phase.

### BRIEF DESCRIPTION OF THE DRAWINGS

FIG. 1: inhibition of the number of immune cells in BALF (#p < 0.01 vs. normal control group, **p < 0.01, *p < 0.05 vs. model control group).
FIG. 2: inhibition of TNFα in BALF (**p < 0.01, *p < 0.05 vs. model control group).

### DETAILED DESCRIPTION

The specific embodiments of the disclose are shown below, and the examples are intended to further describe rather than limit the disclose. Any technical solution equivalent to the disclose falls within the scope of the disclose.

### Example 1

### Preparation of 9,10-dimethoxy-2-[[2-(2-oxo-imidazolidin-1-yl)-ethyl]-(2,4,6-trimethylphenyl)-amino]-6,7-dihydro-pyrimido[6,1-α]isoquinolin-4-one (Compound 1) (see preparation method in WO2022228544)

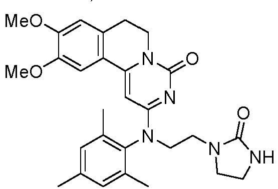

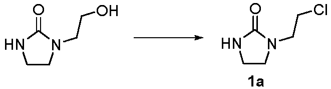

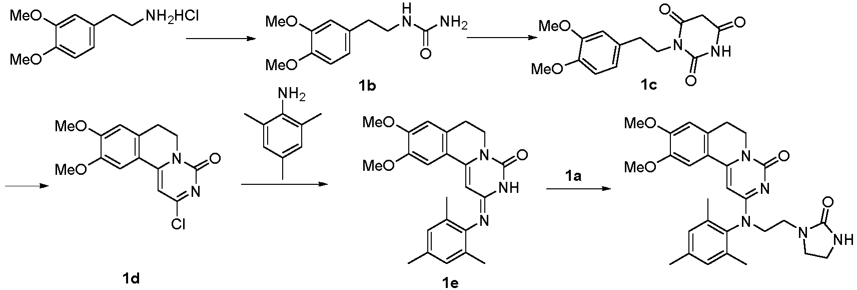

### Preparation of intermediate 1a: 1-(2-chloroethyl)imidazolidin-2-one

Thionyl chloride (5 mL) was slowly added to 1-(2-hydroxyethyl)imidazolidinone (3.5 g, 26.9 mmol) at 0 °C. The mixture was heated to 45 °C and stirred until the reaction was completed. A saturated sodium chloride solution was added to quench the reaction, and the pH was adjusted to 7 with a 10% NaOH solution. The resulting mixture was extracted with dichloromethane, washed with a saturated sodium chloride solution, and dried over anhydrous sodium sulfate. The filtrate was concentrated under reduced pressure to give intermediate 1a (3.5 g, 88.4% yield), MS(ESI) m/z 149.1 [M+H]⁺.

### Preparation of intermediate 1b: 1-(3,4-dimethoxyphenethyl)urea

2-(3,4-Dimethoxyphenyl)ethylamine hydrochloride (4.3 g, 19.8 mmol) was dissolved in water (25 mL) at room temperature, and the solution was heated to 50 °C. Potassium cyanate (1.8 g, 21.8 mmol) was added in batches, and the resulting mixture was stirred until the reaction was completed. The reaction mixture was cooled to 0 °C and filtered. The filter cake was washed with ice-cold water and dried to give intermediate 1b (4.1 g, 93.8% yield), MS(ESI) m/z 225.1 [M+H]⁺.

### Preparation of intermediate 1c: 1-[2-(3,4-dimethoxy-phenyl)-ethyl]-pyrimidine-2,4,6-trione

Sodium ethoxide (3.8 g, 55.8 mmol) was added in batches to absolute ethanol (50 mL) in an ice bath. After the addition, the mixture was heated at reflux, and diethyl malonate (5.9 g, 36.6 mmol) was added dropwise. After the addition, the resulting mixture was stirred for 0.25 h to 0.5 h. A solution of intermediate 1b (4.1 g, 18.3 mmol) in ethanol (30 mL) was added dropwise, and the mixture was stirred until the reaction was completed. The reaction mixture was cooled to 0 °C, and a 5% HCl solution was added dropwise until the pH was 6. Water (300 mL) was added, and the mixture was filtered. The filter cake was washed with ice-cold water and dried to give intermediate 1c (3.9 g, 77.1% yield), MS(ESI) m/z 293.1 [M+H]⁺.

### Preparation of intermediate 1d: 2-chloro-9,10-dimethoxy-6,7-dihydropyrimido[6,1-a]isoquinolin-4-one

Intermediate 1c (3.9 g, 13.4 mmol) was added to phosphorus oxychloride (120 mL) at room temperature. The mixture was heated to 110 °C and stirred until the reaction was completed. The reaction mixture was cooled and concentrated, and the solid was poured into ice-cold water. A saturated NaOH solution was added dropwise until the pH was 10, and the resulting mixture was filtered. The filter cake was washed with ice-cold water and dried to give intermediate 1d (2.4 g, 62.4% yield), MS(ESI) m/z 293.1 [M+H]⁺.

### Preparation of intermediate 1e: 9,10-dimethoxy-2-(2,4,6-trimethyl-phenylimino)-2,3,6,7-tetrahydropyrimido[6,1-a]isoquinolin-4-one

Intermediate 1d (2.4 g, 8.2 mmol) was suspended in isopropanol (30 mL) at room temperature, and 2,4,6-trimethylaniline (4.5 g, 24.6 mmol) was added. The system was heated to 90 °C and stirred until the reaction was completed. The reaction mixture was cooled and filtered. The filter cake was washed with ice-cold water and dried to give intermediate 1e (3.0 g, 92.1% yield), MS(ESI) m/z 392.2 [M+H]⁺.

### Preparation of compound 1: 9,10-dimethoxy-2-[[2-(2-oxo-imidazolidin-1-yl)-ethyl]-(2,4,6-trimethyl-phenyl)-amino]-6,7-dihydro-pyrimido[6,1-a]isoquinolin-4-one

Intermediate 1e (0.72 g, 1.8 mmol) was dissolved in tetrahydrofuran (20 mL) at room temperature, and potassium *tert*-butoxide (0.42 g, 3.6 mmol) was added under a nitrogen atmosphere. After the addition, the mixture was heated to 65 °C, stirred for 48 h, and cooled to 25 °C, and intermediate 1a (0.82 g, 5.5 mmol) was added. After the addition, the resulting mixture was heated to 80 °C and stirred until the reaction was completed. A saturated sodium chloride solution was added to quench the reaction, and the reaction mixture was extracted with dichloromethane, washed with a saturated sodium chloride solution, and dried over anhydrous sodium sulfate. The filtrate was concentrated under reduced pressure and purified by silica gel column chromatography (n-heptane/ethyl acetate) to give the target compound 1 (0.21 g, 46.5% yield).

¹H NMR (400 MHz, CDCl₃) *δ* 6.99 (s, 2H), 6.69 (s, 1H), 6.64 (s, 1H), 5.39 (s, 1H), 4.61 (s, 1H), 4.22-4.15 (m, 2H), 4.08-3.99 (m, 2H), 3.93 (s, 3H), 3.77-3.69 (m, 5H), 3.55-3.46 (m, 2H), 3.38-3.42 (t, J = 6.8 Hz, 2H), 2.88-2.92 (t, J = 6.4 Hz, 2H), 2.34 (s, 3H), 2.18 (s, 6H).

MS(ESI) m/z 504.4 [M+H]⁺.

### Test Example 1: In Vitro PDE4B Enzyme Activity Assay: An IMAP FP-Based Analysis Method

### 1. Materials

| Material | Brand | Cat. No./model |
|---|---|---|
| PDE4B1 | BPS | 60041 |
| Trequinsin | Sigma | T2057 |
| 384-well plate | Perkin Elmer | 6007279 |
| IMAP FP IPP Explorer Kit | MOLECULAR DEVICES | R8124 |

### 2. Procedures

The compound was serially diluted 5-fold with DMSO to different concentrations (10,000 nM, 2000 nM, 400 nM, 80 nM, 16 nM, 3.2 nM, 0.64 nM, 0.128 nM, 0.0256 nM, and 0.005 nM). 200 µL of the compound at different concentrations was added to a 384-well plate (n = 2), and meanwhile, two 200-µL parts of DMSO were added to the 384-well plate (n = 2) as blank controls. Then 10 µL of a 0.025 µg/mL PDE4B1 enzyme solution (prepared with 1 mM 5*IMAP reaction buffer and 1 mM DTT) was added to the 384-well plate, and 10 µL of PDE4B1 enzyme-free blank buffer was added to one of the blank controls. The plate was incubated with shaking at room temperature for 15 min, and then 10 µL of a 0.1 µM FAM-cAMP solution (prepared with 1 mM 5*IMAP reaction buffer and 1 mM DTT) was added to the plate. After the plate was incubated with shaking at room temperature for 30 min, 60 µL of an assay solution (prepared with 0.5625 mM 5*IMAP progressive binding buffer A, 0.1875 mM 5*IMAP progressive binding buffer B, and 0.75 mM beads) was added. After the plate was incubated with shaking at room temperature for 60 min, data were collected. Inhibition rates were calculated by the formula: inhibition rate = M/(M - M_{control}) × 100; the IC₅₀ value was calculated from a fitted curve of concentrations vs. inhibition rates. RPL554 was used as a positive control in this assay.

The *in vitro* inhibition of PDE4B1 enzyme activity by the example of the disclose was assessed through the above assay, and the calculated IC₅₀ was 50 nM.

### Test Example 2: In Vitro PDE3A Enzyme Activity Assay: An IMAP FP-Based Analysis Method

### 1. Materials

| Material | Brand | Cat. No./model |
|---|---|---|
| PDE3A | BPS | 60030 |
| Trequinsin | Sigma | T2057 |
| 384-well plate | Perkin Elmer | 6007279 |
| IMAP FP IPP Explorer Kit | MOLECULAR DEVICES | R8124 |

### 2. Procedures

The compound was serially diluted 5-fold with DMSO to different concentrations (10,000 nM, 2000 nM, 400 nM, 80 nM, 16 nM, 3.2 nM, 0.64 nM, 0.128 nM, 0.0256 nM, and 0.005 nM). 200 µL of the compound at different concentrations was added to a 384-well plate (n = 2), and meanwhile, two 200-µL parts of DMSO were added to the 384-well plate (n = 2) as blank controls. Then 10 µL of a 0.025 µg/mL PDE4B1 enzyme solution (prepared with 1 mM 5*IMAP reaction buffer and 1 mM DTT) was added to the 384-well plate, and 10 µL of PDE3A enzyme-free blank buffer was added to one of the blank controls. The plate was incubated with shaking at room temperature for 15 min, and then 10 µL of a 0.1 µM FAM-cAMP solution (prepared with 1 mM 5*IMAP reaction buffer and 1 mM DTT) was added to the plate. After the plate was incubated with shaking at room temperature for 30 min, 60 µL of an assay solution (prepared with 0.5625 mM 5*IMAP progressive binding buffer A, 0.1875 mM 5*IMAP progressive binding buffer B, and 0.75 mM beads) was added. After the plate was incubated with shaking at room temperature for 60 min, data were collected. Inhibition rates were calculated by the formula: inhibition rate = M/(M - M_{control}) × 100; the IC₅₀ value was calculated from a fitted curve of concentrations vs. inhibition rates. RPL554 was used as a positive control in this assay.

The *in vitro* inhibition of PDE3A enzyme activity by the example of the disclose was assessed through the above assay, and the calculated IC₅₀ was 0.13 nM.

### Example 2

Different formulation formulas were prepared by the following steps using 9,10-dimethoxy-2-[[2-(2-oxo-imidazolidin-1-yl)-ethyl]-(2,4,6-trimethyl-phenyl)-amino]-6,7-dihydro-pyrimido[6,1-*a*]-isoquinolin-4-one (compound A) and the auxiliary materials listed in Table 1.

Step 1: preparation of a buffer: Formula amounts of polysorbate 20, span 20, polysorbate 80, sodium dihydrogen phosphate, disodium hydrogen phosphate, and sodium chloride were weighed out and dissolved by stirring in an appropriate amount of water.

Step 2: A formula amount of compound A was added, and the volume was brought to 1/20 of the total preparation volume with the buffer obtained in step 1. The mixture was uniformly stirred, sheared with a high-speed shear T50 (at a shearing rotational speed of about 5000 rpm) for 2 min to 6 min, and sterilized to obtain a concentrated suspension.

Step 3: The buffer obtained in step 1 was added to the concentrated suspension obtained in step 2, and the mixture was mixed, diluted to volume, and sheared with a high-speed shear T50 for 10 min to 30 min.

Filling was performed. The mixture was aliquoted into polyester/aluminum/polyethylene pharmaceutical composite bags and sealed for storage.

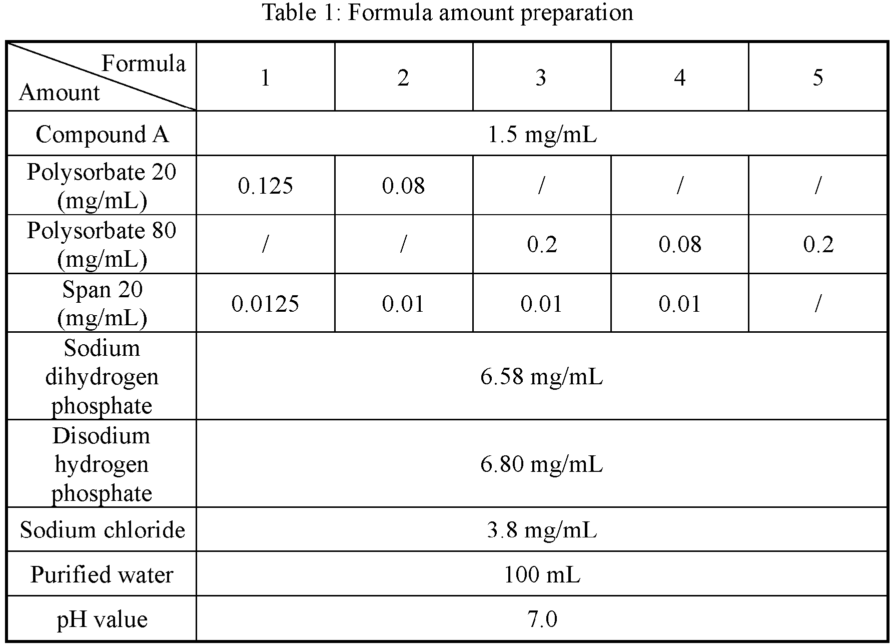

The aforementioned formulas 1-5 were tested for stability under different conditions. The data are shown in Table 2.

**Table 2**

| Batch No. | Conditions | Particle size and particle size distribution (µm) | | | | Compound A content (%) | Total impurity (%) |
|---|---|---|---|---|---|---|---|
| | | D₁₀ | D₅₀ | D₉₀ | D [4, 3] | | |
| Formula 1 | 0d | 0.44 | 1.62 | 2.81 | 1.65 | 93.7 | 1.96 |
| | 40°C7d | 0.77 | 1.75 | 3.08 | 1.86 | 93.7 | 2.02 |
| | 40°C14d | 0.71 | 1.72 | 3.02 | 1.82 | 93.7 | 2.01 |
| | 40°C1M | 0.40 | 1.73 | 2.96 | 1.73 | 92.2 | 2.08 |
| | 40°C2M | 0.75 | 2.08 | 3.71 | 2.18 | 94.8 | 1.95 |
| | 25°C1M | 0.94 | 5.07 | 9.84 | 5.66 | 81.3 | 2.11 |
| | 25°C2M | 1.59 | 6.01 | 11.52 | 7.06 | 79.4 | 1.97 |
| | 25°C3M | 1.37 | 5.51 | 9.98 | 5.80 | N/A | 2.00 |
| Formula 2 | 0d | 0.55 | 1.69 | 2.93 | 1.75 | 88.5 | 1.97 |
| | 40°C7d | 0.75 | 1.73 | 3.01 | 1.83 | 89.9 | 1.99 |
| | 40°C14d | 0.77 | 1.88 | 3.25 | 1.97 | 89.7 | 1.99 |
| | 40°C1M | 0.74 | 2.04 | 3.59 | 2.13 | 89.3 | 2.04 |
| | 40°C2M | 0.69 | 2.91 | 5.33 | 3.02 | 89.7 | 1.98 |
| | 25°C1M | 2.30 | 6.77 | 12.80 | 7.50 | 81.4 | 2.09 |
| | 25°C2M | 1.12 | 6.23 | 11.67 | 6.65 | 79.1 | 2.04 |
| | 25°C3M | 1.12 | 6.30 | 11.73 | 6.69 | 81.1 | 2.04 |
| Formula 3 | 0d | 0.34 | 1.47 | 3.34 | 1.68 | 96.1 | 2.03 |
| | 40°C7d | 0.50 | 1.64 | 2.83 | 1.68 | 95.6 | 1.99 |
| | 40°C14d | 0.37 | 1.56 | 3.18 | 1.69 | 95.9 | 2.00 |
| | 40°C1M | 0.45 | 1.61 | 2.74 | 1.63 | 96.6 | 2.02 |
| | 40°C2M | 0.41 | 1.59 | 2.96 | 1.67 | 95.2 | 1.92 |
| | 25°C1M | 0.42 | 1.58 | 2.97 | 1.67 | 94.8 | 2.07 |
| | 25°C2M | 0.44 | 1.62 | 2.76 | 1.64 | 96.5 | 1.99 |
| | 25°C3M | 0.36 | 1.53 | 3.41 | 1.73 | 94.2 | 1.99 |
| Formula 4 | 0d | 0.31 | 1.39 | 3.18 | 1.58 | 92.2 | 1.96 |
| | 40°C7d | 0.46 | 1.59 | 2.71 | 1.62 | 93.8 | 1.99 |
| | 40°C14d | 0.36 | 1.52 | 3.35 | 1.71 | 96.3 | 1.98 |
| | 40°C1M | 0.37 | 1.53 | 3.09 | 1.65 | 94.3 | 2.04 |
| | 40°C2M | 0.48 | 1.67 | 2.86 | 1.70 | 94.2 | 1.97 |
| | 25°C1M | 0.42 | 1.58 | 2.66 | 1.58 | 93.6 | 2.03 |
| | 25°C2M | 0.45 | 1.61 | 2.72 | 1.62 | 93.7 | 1.99 |
| | 25°C3M | 0.35 | 1.46 | 3.25 | 1.65 | 93.5 | 2.00 |
| Formula 5 | 0d | 0.39 | 1.58 | 2.95 | 1.65 | 94.4 | 2.01 |
| | 40°C7d | 0.69 | 1.70 | 2.94 | 1.78 | 95.4 | 2.00 |
| | 40°C14d | 0.57 | 1.66 | 2.84 | 1.71 | 96.0 | 2.01 |
| | 40°C1M | 0.40 | 1.61 | 2.73 | 1.61 | 93.8 | 2.05 |
| | 40°C2M | 0.50 | 1.66 | 2.86 | 1.70 | 92.7 | 1.96 |
| | 25°C1M | 0.67 | 1.58 | 2.73 | 1.67 | 94.3 | 2.08 |
| | 25°C2M | 0.35 | 1.56 | 3.21 | 1.69 | 95.5 | 1.97 |
| | 25°C3M | 0.42 | 1.60 | 2.74 | 1.61 | 93.6 | 1.95 |

| | | | | | | | |
|---|---|---|---|---|---|---|---|
| Note: d represents day, and M represents month. | | | | | | | |

Conclusion: According to the General Chapter on Inhalation Formulations in Volume IV of the Chinese Pharmacopoeia, the particle size of drugs in inhalations should generally be controlled below 10 µm, with most particles being below 5 µm.Under long-term storage or accelerated storage conditions, the particle size of the active ingredient in the compositions of formula 1 and formula 2 increased significantly, with the D90 value exceeding 5 µm and even exceeding 10 µm.Compared with the formulas containing polysorbate 20, the formulas containing polysorbate 80 maintained stable particle sizes under accelerated conditions or long-term storage at 25 °C, demonstrating better storage stability of the compositions.

A breathing simulator (model: Copley BRS2100) and a nebulizer pump (model: PARI TurboBOY) were used to simulate adult breathing (simulation parameters: tidal volume: 500 mL; frequency of breathing: 15 cycles/min; breathing waveform: sinusoidal; inhalation-to-exhalation ratio: 1:1), and the FPF (fine particle fraction) of formula 5 was measured to be 52.64%.

### Example 3

Different formulation formulas were prepared by the steps of Example 2 using 9,10-dimethoxy-2-[[2-(2-oxo-imidazolidin-1-yl)-ethyl]-(2,4,6-trimethyl-phenyl)-amino]-6,7-dihydro-pyrimido[6,1-*a*]-isoquinolin-4-one (compound A) and the auxiliary materials listed in Table 3.

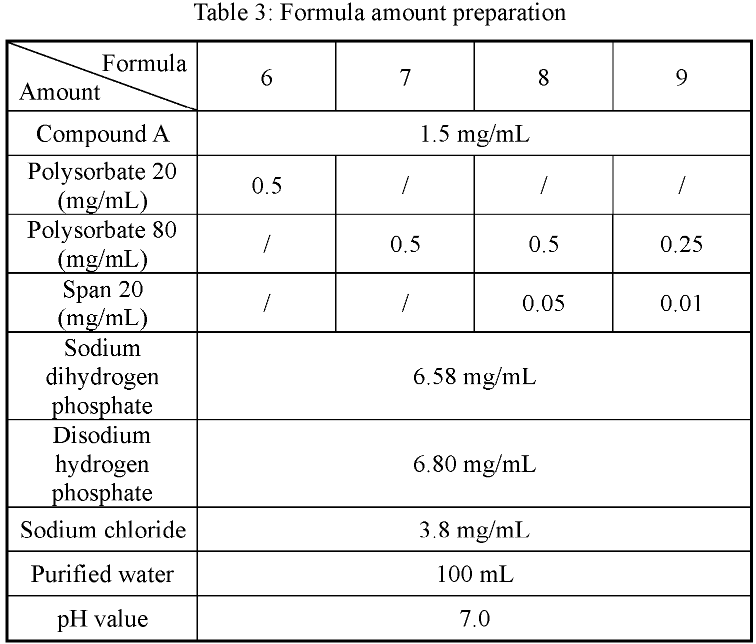

The aforementioned formulas 6-9 were tested for stability under different conditions. The data are shown in Table 4.

**Table 4**

| Batch No. | Conditions | Particle size and particle size distribution (µm) | | | |
|---|---|---|---|---|---|
| | | D₁₀ | D₅₀ | D₉₀ | D [4, 3] |
| Formula 6 | 0d | 0.76 | 1.97 | 3.41 | 2.05 |
| | 40°C7d | 0.75 | 2.08 | 3.64 | 2.17 |
| | 40°C14d | 0.66 | 2.56 | 4.76 | 2.68 |
| | 40°C1M | 0.69 | 2.66 | 4.88 | 2.77 |
| | 40°C2M | 0.66 | 2.44 | 4.48 | 2.53 |
| | 25°C1M | 0.75 | 2.07 | 3.64 | 2.16 |
| | 25°C2M | 0.72 | 1.97 | 3.43 | 2.04 |
| | 25°C3M | 0.73 | 2.03 | 3.54 | 2.11 |
| Formula 7 | 0d | 0.42 | 1.62 | 2.78 | 1.63 |
| | 40°C7d | 0.42 | 1.61 | 2.74 | 1.62 |
| | 40°C14d | 0.76 | 1.83 | 3.14 | 1.91 |
| | 40°C1M | 0.72 | 1.82 | 3.17 | 1.90 |
| | 40°C2M | 0.57 | 1.75 | 3.04 | 1.81 |
| | 25°C1M | 0.64 | 1.71 | 2.92 | 1.77 |
| | 25°C2M | 0.51 | 1.66 | 2.83 | 1.70 |
| | 25°C3M | 0.77 | 1.93 | 3.31 | 2.01 |
| Formula 8 | 0d | 0.35 | 1.43 | 3.22 | 1.63 |
| | 40°C7d | 0.60 | 1.56 | 3.32 | 3.30 |
| | 40°C14d | 0.31 | 1.46 | 3.34 | 1.66 |
| | 40°C1M | 0.38 | 1.53 | 3.11 | 1.66 |
| | 40°C2M | 0.35 | 1.52 | 3.38 | 1.71 |
| | 25°C1M | 0.37 | 1.54 | 3.10 | 1.66 |
| | 25°C2M | 0.32 | 1.47 | 3.30 | 1.66 |
| | 25°C3M | 0.34 | 1.48 | 3.32 | 1.68 |
| Formula 9 | 0d | 0.36 | 1.48 | 3.33 | 1.69 |
| | 40°C7d | 0.75 | 1.69 | 2.95 | 1.79 |
| | 40°C14d | 0.77 | 1.83 | 3.15 | 1.92 |
| | 40°C1M | 0.76 | 1.75 | 3.05 | 1.85 |
| | 40°C2M | 0.73 | 1.75 | 3.06 | 1.85 |
| | 25°C1M | 0.54 | 1.71 | 2.94 | 1.76 |
| | 25°C2M | 0.69 | 1.78 | 3.05 | 1.85 |
| | 25°C3M | 0.63 | 1.66 | 2.86 | 1.73 |

### Example 4

Step 1: preparation of a buffer: Formula amounts of polysorbate 80, sodium dihydrogen phosphate, disodium hydrogen phosphate, and sodium chloride were weighed out and dissolved by stirring in an appropriate amount of water.

Step 2: A formula amount of compound A was added, and the volume was brought to 1/20 of the total preparation volume with the buffer obtained in step 1. The mixture was uniformly stirred, sheared with a high-speed shear T50 (at a shearing rotational speed of about 5000 rpm) for 2 min to 6 min, and sterilized to obtain a concentrated suspension. Step 3: The buffer obtained in step 1 was added to the concentrated suspension obtained in step 2, and the mixture was mixed, diluted to volume, and sheared with a high-speed shear T50 for 10 min to 30 min.

**Table 5**

| Formula | Amount | |
|---|---|---|
| | Formula 10 | Formula 11 |
| Compound A | 37.5 mg | 600 mg |
| Polysorbate 80 | 20 mg | 50 mg |
| Sodium dihydrogen phosphate | 269 mg | 269 mg |
| Disodium hydrogen phosphate | 376 mg | 376 mg |
| Sodium chloride | 716 mg | 716 mg |
| Purified water | 100 mL | 100 mL |

| | | |
|---|---|---|
| Note: The batch size was 20,000 units. | | |

The aforementioned formulas were tested for stability at 40 °C ± 2 °C/25% RH ± 5% RH. The data are shown in Table 6.

**Table 6**

| Formula | Time point | Particle size and particle | | | Compound A content (%) | Total impurity (%) | Polysorbate 80 Content (%) (mg/mL) |
|---|---|---|---|---|---|---|---|
| | | D₁₀ | D₅₀ | D₉₀ | | | |
| Formula 11 | 0d | 0.76 | 1.97 | 3.40 | 98.6 | 2.33 | 0.21 |
| | 40°C14d | 0.71 | 2.28 | 4.11 | 100.3 | 2.24 | 0.19 |
| | 40°C1M | 0.72 | 2.30 | 4.13 | 99.7 | 2.17 | 0.21 |
| Formula 12 | 0d | 0.71 | 1.92 | 3.37 | 97.0 | 2.21 | 0.22 |
| | 40°C14d | 0.72 | 1.86 | 3.30 | 95.8 | 2.16 | 0.23 |
| | 40°C1M | 0.72 | 2.06 | 3.62 | 97.6 | 2.21 | 0.24 |

### Example 5: Efficacy Study in Mouse Model of LPS-Induced Acute Pulmonary Inflammation

The study utilized a BALB/c mouse model of LPS-induced acute pulmonary inflammation to evaluate the inhibitory effect of the compound on pulmonary inflammation based on immune cell counts and TNFα cytokine levels in bronchoalveolar lavage fluid (BALF). The mice were divided into 6 groups, with 8 mice per group, including: a normal control group, a model control group, an RPL554 group (1.0 mg/kg), and low, medium, and high dose groups of compound A (0.3 mg/kg, 1.0 mg/kg, and 3.0 mg/kg). One hour after intratracheal administration of nebulized drugs, the mice were stimulated by nebulized LPS for modeling. Six hours after modeling, BALF was collected from the animals, and the total cells, the neutrophils, and the eosinophils in BALF were counted, along with the measurement of the pro-inflammatory cytokine TNFα level in BALF.

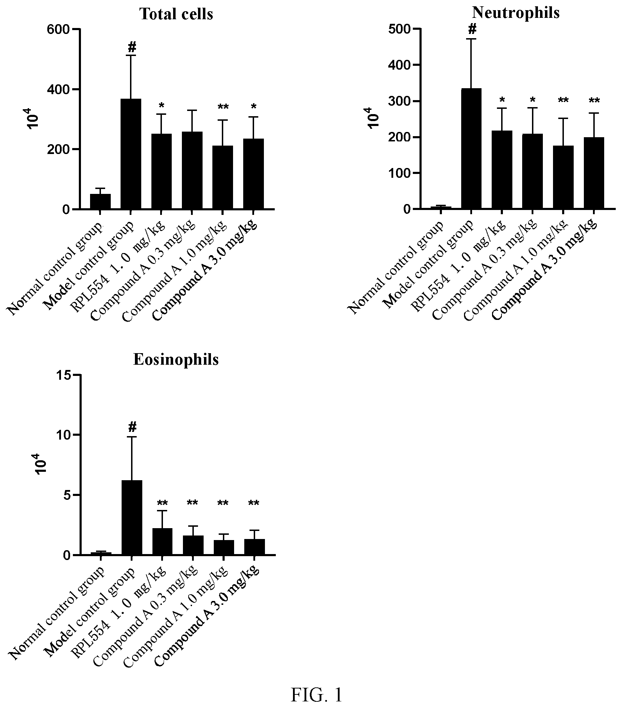

The cell counts in BALF are shown in FIG. 1. The numbers of total cells, neutrophils, and eosinophils in BALF in the model control group were significantly higher than those in the normal control group. After administration at 1.0 mg/kg, the numbers of total cells, neutrophils, and eosinophils in BALF in the RPL554 positive control group were significantly lower than those in the model group. The numbers of neutrophils and eosinophils in each of the compound A 0.3 mg/kg, 1.0 mg/kg, and 3.0 mg/kg groups were also significantly lower than those in the model group, and compound A at 0.3 mg/kg was comparable in efficacy to RPL554 at 1 mg/kg; the number of total cells in each of the 1.0 mg/kg and 3.0 mg/kg groups was significantly lower, and the 0.3 mg/kg group showed a downward trend.

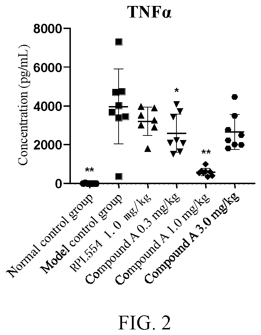

The TNFα levels in BALF are shown in FIG. 2. The TNFα level in BALF in the model control group was significantly higher than that in the normal control group. After administration at 1.0 mg/kg, the RPL554 positive control group showed a downward trend in the TNFα level in BALF compared with the model group. All the doses of compound A reduced TNFα levels, and the TNFα levels in the 0.3 mg/kg and 1.0 mg/kg groups were significantly lower than that in the model group.

### Example 6

9,10-Dimethoxy-2-[[2-(2-oxo-imidazolidin-1-yl)-ethyl]-(2,4,6-trimethyl-phenyl)-amino]-6,7-dihydro-pyrimido[6,1-*a*]-isoquinolin-4-one (compound A), sodium carboxymethyl cellulose, polysorbate 20, sodium dihydrogen phosphate, disodium hydrogen phosphate, and sodium chloride were weighed out according to Table 7, and formulation compositions were prepared by the following steps.

**Table 7**

| Formula | Amount | | |
|---|---|---|---|
| | Formula 12 | Formula 13 | Formula 14 |
| Compound A | | 1.5 mg/mL | |
| Polysorbate 20 | 10 mg/mL | 0.2 mg/mL | 0.5 mg/mL |
| Sodium carboxymethyl cellulose | 10 mg/mL | 0.2 mg/mL | 0.5 mg/mL |
| Sodium dihydrogen phosphate | | 6.58 mg/mL | |
| Disodium hydrogen phosphate | | 6.80 mg/mL | |
| Sodium chloride | | 3.8 mg/mL | |
| Purified water | | 100 mL | |
| pH value | | 7.0 | |

Step 1: preparation of a buffer: Formula amounts of polysorbate 20, sodium dihydrogen phosphate, disodium hydrogen phosphate, and sodium chloride were weighed out and dissolved by stirring in an appropriate amount of water.

Step 2: A formula amount of compound A was added, and the volume was brought to 1/20 of the total preparation volume with the buffer obtained in step 1. The mixture was uniformly stirred, sheared with a high-speed shear T50 (at a shearing rotational speed of about 5000 rpm) for 2 min to 6 min, and sterilized to obtain a concentrated suspension. Step 3: The buffer obtained in step 1 was added to the concentrated suspension obtained in step 2, and the mixture was mixed, diluted to volume, and sheared with a high-speed shear T50 for 10 min to 30 min.

The aforementioned formulas 12-14 were tested for stability under different conditions. The data are shown in Table 8.

**Table 8**

| Batch No. | Conditions | Particle size and particle size distribution (µm) | | | | | |
|---|---|---|---|---|---|---|---|
| | | D₁₀ | D₅₀ | D₉₀ | D [4, 3] | Compound A content (%) | Total impurity (%) |
| Formula 12 | 0d | 0.75 | 1.87 | 3.30 | 1.97 | 103.1 | 2.3 |
| | 40°C7d | 0.71 | 2.01 | 3.65 | 2.12 | 100.6 | 2.2 |
| | 40°C14d | 0.88 | 3.28 | / | / | 101.3 | 2.2 |
| | 40°C1M | 0.76 | 1.99 | 3.46 | 2.08 | 100.5 | 2.4 |
| | 40°C2M | 0.77 | 2.09 | 3.74 | 2.20 | 99.4 | 2.2 |
| | 25°C1M | 0.75 | 2.01 | 3.88 | 2.78 | 104.9 | 2.6 |
| | 25°C2M | 0.52 | 1.94 | 3.39 | 1.99 | 103.1 | 2.3 |
| | 25°C3M | 0.68 | 2.09 | 5.42 | 3.63 | 105.2 | 2.5 |
| Formula 13 | 0d | 0.74 | 2.01 | 3.49 | 2.08 | 98.1 | 2.2 |
| | 40°C7d | 0.80 | 4.48 | 9.29 | 12.76 | 97.3 | 2.2 |
| | 40°C14d | 0.77 | 4.32 | 8.53 | 4.63 | 94.5 | 2.1 |
| | 40°C1M | 0.79 | 4.33 | 8.40 | 4.63 | 90.8 | 2.2 |
| | 40°C2M | 0.90 | 4.89 | 9.32 | 5.21 | 92.3 | 1.7 |
| | 25°C1M | 0.80 | 4.39 | 8.25 | 4.61 | 92.5 | 2.2 |
| | 25°C2M | 0.74 | 3.98 | 7.53 | 4.18 | 92.2 | 2.0 |
| | 25°C3M | 0.71 | 3.59 | 6.71 | 3.75 | 96.3 | 2.3 |
| Formula 14 | 0d | 0.74 | 2.00 | 3.48 | 2.08 | 106.0 | 2.4 |
| | 40°C7d | 0.48 | 1.98 | 3.42 | 2.01 | 105.4 | 2.4 |
| | 40°C14d | 0.56 | 1.96 | 3.41 | 2.01 | 104.9 | 2.3 |
| | 40°C1M | 0.45 | 1.97 | 3.40 | 1.98 | 106.2 | 1.9 |
| | 40°C2M | 0.72 | 2.08 | 3.63 | 2.16 | 103.2 | 2.1 |
| | 25°C1M | 0.52 | 1.97 | 3.39 | 2.00 | 106.0 | 2.4 |
| | 25°C2M | 0.69 | 2.74 | 5.15 | 2.88 | 104.6 | 2.1 |
| | 25°C3M | 0.72 | 3.40 | 6.53 | 3.60 | 106.3 | 2.4 |

| | | | | | | | |
|---|---|---|---|---|---|---|---|
| Note: / indicates that the data for the batch showed significant variability and were inaccurate. | | | | | | | |

A breathing simulator (model: Copley BRS2100) and a nebulizer pump (model: PARI TurboBOY) were used to simulate adult breathing (simulation parameters: tidal volume: 500 mL; frequency of breathing: 15 cycles/min; breathing waveform: sinusoidal; inhalation-to-exhalation ratio: 1:1), and the FPF of formula 12 was measured to be 44.1%.

## Claims

1. A pharmaceutical composition, comprising 9,10-dimethoxy-2-[[2-(2-oxo-imidazolidin-1-yl)-ethyl]-(2,4,6-trimethyl-phenyl)-amino]-6,7-dihydro-pyrimido[6,1-*a*]-isoquinolin-4-one or a pharmaceutically acceptable salt thereof as an active ingredient, and polysorbate 80.

2. The pharmaceutical composition according to claim 1, wherein the active ingredient is at a concentration of 0.01 mg/mL to 40 mg/mL, preferably 0.05 mg/mL to 20 mg/mL.

3. The pharmaceutical composition according to claim 1 or 2, further comprising a buffer, wherein the buffer is preferably a citrate buffer or a phosphate buffer or an acetate buffer, more preferably a phosphate buffer, and most preferably a sodium dihydrogen phosphate-disodium hydrogen phosphate buffer.

4. The pharmaceutical composition according to any one of claims 1-3, wherein the polysorbate 80 is at a concentration of 0.05 mg/mL to 5.0 mg/mL, preferably 0.08 mg/mL to 0.5 mg/mL.

5. The pharmaceutical composition according to any one of claims 1-4, wherein the buffer has a concentration of 1 mM to 50 mM, preferably 5 mM to 30 mM.

6. The pharmaceutical composition according to any one of claims 1-5, wherein the composition has a pH of 3.0 to 8.0.

7. The pharmaceutical composition according to any one of claims 1-6, further comprising a sorbitan fatty acid ester.

8. The pharmaceutical composition according to any one of claims 1-7, wherein the active ingredient is present as particles having a particle size D90 of less than 7 µm, preferably 1.0 µm to 5.0 µm, such as 3.0 µm or 3.2 µm.

9. A pharmaceutical composition, comprising:
a) 0.05 mg/mL to 40 mg/mL 9,10-dimethoxy-2-[[2-(2-oxo-imidazolidin-1-yl)-ethyl]-(2,4,6-trimethyl-phenyl)-amino]-6,7-dihydro-pyrimido[6,1-*a*]-isoquinolin-4-one or a pharmaceutically acceptable salt thereof as an active ingredient,
b) 0.05 mg/mL to 2.0 mg/mL polysorbate 80, and
c) a 1 mM to 50 mM buffer.

10. A pharmaceutical composition, comprising particles of 9,10-dimethoxy-2-[[2-(2-oxo-imidazolidin-1-yl)-ethyl]-(2,4,6-trimethyl-phenyl)-amino]-6,7-dihydro-pyrimido[6,1-*a*]-isoquinolin-4-one or a pharmaceutically acceptable salt thereof as an active ingredient, wherein the active ingredient particles have a particle size D90 of less than 7 µm, preferably 1.0 µm to 5.0 µm, such as 3.0 µm or 3.2 µm.

11. The pharmaceutical composition according to any one of claims 1-10, further comprising a tonicity modifier, wherein the tonicity modifier is preferably sodium chloride.

12. The pharmaceutical composition according to any one of claims 1-11, wherein the pharmaceutical composition is suitable for administration by a nebulizer.

13. A method for preparing the pharmaceutical composition according to any one of claims 1-12, comprising a step of grinding, wet grinding, homogenizing, precipitating, or shearing the active ingredient 9,10-dimethoxy-2-[[2-(2-oxo-imidazolidin-1-yl)-ethyl]-(2,4,6-trimethyl-phenyl)-amino]-6,7-dihydro-pyrimido[6,1-*a*]-isoquinolin-4-one or the pharmaceutically acceptable salt thereof.

14. A method for preparing a freeze-dried formulation comprising 9,10-dimethoxy-2-[[2-(2-oxo-imidazolidin-1-yl)-ethyl]-(2,4,6-trimethyl-phenyl)-amino]-6,7-dihydro-pyrimido[6,1-*a*]-isoquinolin-4-one or a pharmaceutically acceptable salt thereof, comprising a step of lyophilizing the pharmaceutical composition according to any one of claims 1-12.

15. A freeze-dried formulation comprising 9,10-dimethoxy-2-[[2-(2-oxo-imidazolidin-1-yl)-ethyl]-(2,4,6-trimethyl-phenyl)-amino]-6,7-dihydro-pyrimido[6,1-*a*]-isoquinolin-4-one or a pharmaceutically acceptable salt thereof prepared by the method according to claim 14.

16. A method for preparing a reconstituted solution comprising 9,10-dimethoxy-2-[[2-(2-oxo-imidazolidin-1-yl)-ethyl]-(2,4,6-trimethyl-phenyl)-amino]-6,7-dihydro-pyrimido[6,1-*a*]-isoquinolin-4-one or a pharmaceutically acceptable salt thereof, comprising a step of reconstituting the freeze-dried formulation according to claim 15, wherein a liquid medium used for reconstitution is preferably water for injection.

17. A reconstituted solution comprising 9,10-dimethoxy-2-[[2-(2-oxo-imidazolidin-1-yl)-ethyl]-(2,4,6-trimethyl-phenyl)-amino]-6,7-dihydro-pyrimido[6,1-*a*]-isoquinolin-4-one or a pharmaceutically acceptable salt thereof prepared by the method according to claim 16.

18. A nebulizer, comprising the pharmaceutical composition according to any one of claims 1-12, the freeze-dried formulation according to claim 15, or the reconstituted solution according to claim 17.

19. Use of the pharmaceutical composition according to any one of claims 1-12, the freeze-dried formulation according to claim 15, or the reconstituted solution according to claim 17 in preparing a medicament for preventing and/or treating asthma, obstructive pulmonary disease, sepsis, nephritis, diabetes, allergic rhinitis, allergic conjunctivitis, ulcerative enteritis, or rheumatism, preferably obstructive pulmonary disease.
